# EUROPEAN PATENT APPLICATION

(11) **EP 1 454 621 A2**
(43) Date of publication of application: **08.09.2004**
(21) Application number: 04075664.5
(22) Date of filing: 03.03.2004
(51) Int. Cl.: A61K 7/48, A61K 7/02, A61K 7/027

(54) **Cosmetic composition containing polyisoprene**

(30) Priority: 04.03.2003 IT MI20030385
(71) Applicant: Intercos S.p.A., 20122 Milano (IT)
(72) Inventor: Maio, Giuseppe, 20080 Zelo Surrigone MI (IT); Rando, Pietro, 20090 Opera MI (IT)
(74) Representative: Mittler, Enrico

(57) **Abstract**

The present invention relates to a cosmetic composition particularly for make-up of facial skin, lips and eyelashes. It contains as essential ingredients a polyisoprene with a molecular weight of between 100,000 and 4,000,000, an oleophilic modified clay, and an organic solvent. The balance contains usual cosmetic excipients, colourants and other cosmetic additives. By the use of the particular polyisoprene improved film-forming properties of the composition are obtained, and the composition is transfer-resistant and easy to apply.

## Description

The present invention relates to a cosmetic composition containing polyisoprene and to a process for preparing the same.

More particularly it relates to a cosmetic composition containing a polyisoprene with a molecular weight of between 100,000 and 4,000,000 and to a process for preparing the same.

Cosmetic products for make-up of face, lips, eyelashes etc often suffer from the drawback that, when they come into contact with e.g. the fingers or clothing, they tend to smudge or soil these surfaces. In addition, in some cases the make-up can also appear to be not homogeneous. Consequently, in order to avoid these problems cosmetic products which have high adhesive properties and which provide for the deposition of a homogeneous, long-lasting film onto e.g. the facial skin, the lips, the eyelashes etc are of particular interest.

In the prior art these problems have been recognized and proposals have been made to reduce these problems. Thus, in US-A-5,948,393 (Tomomasa, et al.) a long-lasting cosmetic composition is described which contains inter alia an oil-soluble film-forming resin. This resin can be one of many types, among which is also mentioned polyisoprene. No specification of the molecular weight of the polyisoprene is given. In US-A-6,471,983 (Veeger, et al.) a cosmetic composition for skin is described which contains a polyisoprene latex, but no specification of the molecular weight of the polyisoprene latex is given. In US-A-4,122,023 (Yasui, et al.) a synthetic saturated oil for lubricants and cosmetics is described which is prepared from a hydrogenated polyisoprene with a low molecular weight of between 290 and 3,000. In US-A-6,312,672 (Coolbaugh, et al.) a sunscreen composition is described which contains a polymer or copolymer of isoprene, butadiene and styrene. Selectively hydrogenated isoprene/butadiene copolymers are preferred. Polymers of conjugated dienes may also be used, and they may be partially, selectively or completely hydrogenated. Their molecular weight may range from 5,000 to 35,000, with a maximum of 50,000.

None of these references describe the use of an unhydrogenated polyisoprene with a molecular weight of between 100,000 and 4,000,000 in cosmetic products, nor the use of an oleophilic modified clay in such products.

The Applicant has found that the use of (un)hydrogenated polyisoprene with a low molecular weight in cosmetic make-up products does not provide for optimum adhesive and long-lasting film-forming and non-transfer properties when applied to e.g. the skin, the lips, the eyelashes etc. In particular, this (un)hydrogenated low molecular weight polyisoprene proved to be unsuitable for application to the lips, as the film tended to crack down under the normal and continuous movement of the lips.

Thus, in the light of the above state of the art, an object of the present invention is to provide a cosmetic composition having high film-forming and adhesive properties together with good sensorial properties and an improved level of comfort.

According to the present invention, this object is achieved by a cosmetic composition comprising polyisoprene with a molecular weight of between 100,000 and 4,000,000, an oleophilic modified clay, an organic solvent, the balance comprising the usual cosmetic excipients, colourants and other additives

The use of polyisoprene according to the present invention enables the realization of a cosmetic product with transfer-resistant properties and unique film-forming and rheological characteristics without impairing its application comfort. Particularly when combined with an oleophilic modified clay, the applicability is improved and when further combined with waxes and/or silicones (and/or derivatives thereof), the film-forming properties and ease of application is enhanced.

Polyisoprene has a molecular weight of between 100,000 and 4,000,000. Preferably the molecular weight ranges from 2,000,000 to 4,000,000.The amount of the polyisoprene used in the composition ranges from 2-25% by weight of the composition.

Polyisoprene is commercially available, e.g. from Kraton Polymers under the trademark Kraton-IR.A preferred polyisoprene is Kraton IR-310, which has a molecular weight of about 3,000,000. This polyisoprene may be used as such in the composition of the present invention, or it may be used as starting material for obtaining a polyisoprene with a lower molecular weight within the range of the present invention. This can be done, for example, by comminuting solid Kraton material and subsequently degrading or depolymerizing it to the required molecular weight. This degrading or depolymerizing can be done in ways, known in the art, by e.g. treating the polyisoprene with a particular depolymerization catalyst, or by mechanically treating the comminuted polyisoprene material in high speed blade mixers to degrade and depolymerize the comminuted material to the required molecular weight within the range of the present invention. In such a way a polyisoprene with a molecular weight of 100,000 can be made.

The molecular weight can be measured by means of Size Exclusion Chromatography (SEC) or by means of Gel Permeation Chromatography (GPC), a method which uses High Performance Layer Chromatography (HPLC) with an isocratic pump, a refraction index detector and a column thermostat at 25° C. The measurement is performed by dissolving 10-50mg of the polyisoprene sample in 1 ml tetrahydrofurane at room temperature. Subsequently, with a suitable column (e.g. from Polymer Laboratories Plgel) and the calibration curve as function of the expected molecular weight, the chromatographic conditions set up during the calibration phase are maintained (tetrahydrofurane mobile phase at room temperature 1ml/min).Once the test samples are injected, the results are evaluated with suitable GPC software to extrapolate the molecular weight.

The oleophilic modified clay that is used in the present invention is a clay, which has been made oleophilic by treating it with a cationic compound. Such clays are known in the art. Typical examples are smectite clays such as hectorites, montmorillonites and bentonites, which have been made oleophilic by treating them with an organic cationic compound. Typical examples of the oleophilic modified clays are stearalkonium bentonite, and preferably disteardimonium hectorite.

The amount of the oleophilic modified clay, used in the present invention, ranges from 0.05- 20% by weight of the composition, and preferably from 0.1-10% by weight.

The organic solvent that is used in the present invention can be any organic solvent, suitable for use in cosmetic products. Typical examples are aliphatic hydrocarbons with 12-22 carbon atoms, such as isoparaffins like isohexane, isododecane, Isopars(RTM) ex Exxon, etc. Isododecane is the preferred solvent.

The solvent is used in the present invention in an amount of between 1.1 - 90% by weight of the composition, preferably 10-80% by weight of the composition.

Preferably, the composition of the invention also comprises a wax, such as candelilla wax, camauba wax, beeswax, ceresine, microcrystalline wax, paraffin wax, silicon wax, polyethylene wax and the like in an amount of between 0.5 - 20% by weight of the composition. Furthermore, the composition may also preferably contain a silicone or derivative thereof, such as (cyclo)polysiloxanes e.g. cyclomethicone and/or dimethicone or derivatives thereof, in an amount of between 0.5 - 20% by weight of the composition.

The balance of the composition contains the usual cosmetic excipients, colourants and other additives in an amount of between 1.1 % -90%, preferably 10-80% by weight of the composition.. Suitable cosmetic excipients are e.g. talc, mica, kaolin, unmodified clays, zinc oxide, calcium carbonate, magnesium carbonate phosphate, calcium bisulphate, starch and its derivatives,nylon, polyethylene, acrylic (co)polymers and so on.

Suitable colourants are e.g. iron oxides, chromium oxide and/or hydroxide, blue and pink ultramarine, manganese violet, titanium dioxide, pearlescent pigments based on mica or bismuth oxychloride substrates, carmin, lakes and pigments based on organic colourants as listed by the CTFA.

Lipophilic (co)polymers derived from e.g. polyvinylpyrrolidone, from fluor-containing monomers, from acrylic monomers etc, as well as lipophilic polyurethane (co)polymers may also be used in the composition of the invention in an amount of between 1 - 20% by weight of the composition. These lipophilic (co)polymers may even enhance the film-forming action of polyisoprene.

Fragrances, preservatives, alcohol, flavours, vitamins, antioxidants, vegetable oils and fats, silicas and other ingredients usually used in cosmetic products may also be used in the composition of the invention. The composition of the invention may be in liquid, semiliquid, paste-like or cake- or other solid form.

The composition of the invention may be made in any convenient way. A suitable way is first to prepare a dispersion of the (if necessary first comminuted and depolymerized) polyisoprene in the organic solvent, and subsequently adding to the resulting semiliquid mixture the other components of the composition.

The invention will be further illustrated by the following non-limitative Examples.

Examples 1-3 are products for make-up of lips; Example 4 is a facial make-up product, and Examples 5 and 6 are mascara and eye-liner products. Examples 7 and 8 are make-up pencil products, and Examples 9 and 10 are make-up products for the lips.

In all examples the polyisoprene used was obtained from the commercial product Kraton IR-310,as described above.

### EXAMPLES

| Example 1 | |
|---|---|
| Ingredient | % weight |
| Isododecane | 61.00 |
| Polyisoprene | 10.00 |
| Disteardimonium Hectorite | 8.50 |
| Propilene Carbonate | 2.00 |
| Alcohol | 0.80 |
| D&C Red 7 Ca Lake | 0.70 |
| Titanium Dioxide | 1.40 |
| Iron Oxide Yellow | 1.15 |
| Fd&C Blue 1 Al Lake | 0.50 |
| Iron Oxide Red | 0.85 |
| Silica | 0.30 |
| Dimethicone | 10.00 |
| Mica and Titanium Dioxide | 2.60 |
| Flavour | 0.20 |
| TOTAL | 100.00 |

| Example 2 | |
|---|---|
| Ingredient | % weight |
| Isododecane | 75.45 |
| Polyisoprene | 8.00 |
| Disteardimonium Hectorite | 6.00 |
| Propylene Carbonate | 1.50 |
| Alcohol | 0.50 |
| D&C Red 7 Ca Lake | 0.70 |
| Titanium Dioxide | 1.40 |
| Iron Oxide Yellow | 1.15 |
| Fd&C Blue 1 Al Lake | 0.40 |
| Iron Oxide Red | 0.85 |
| Silica | 0.30 |
| Mica and Titanium Dioxide | 2.60 |
| Flavour | 0.10 |
| Tocopheryl Linoleate | 0.05 |
| TOTAL | 100.00 |

| Example 3 | |
|---|---|
| Ingredient | % weight |
| Isododecane | 78.222 |
| Polyisoprene | 8.000 |
| C30-38 Olefin/Isopropyl Maleate/Ma Copolymer | 5.000 |
| Titanium Dioxide | 2.355 |
| Mica | 2.145 |
| Disteardimonium Hectorite | 1.640 |
| Propylene carbonate | 0.380 |
| Iron Oxide Yellow | 0.950 |
| Iron Oxide Red | 0.800 |
| D&C Red 7 Ca Lake | 0.240 |
| Flavour | 0.200 |
| Fd&C Blue 1 Al Lake | 0.068 |
| TOTAL | 100.00 |

| Example 4 | |
|---|---|
| Ingredient | % weight |
| Isohexadecane | 26.0 |
| Isododecane | 22.5 |
| Polyisoprene | 2.00 |
| Polyethylene | 7.50 |
| Euphorbia Cerifera (Candelilla) Wax | 1.00 |
| Bis-Diglyceryl Polyacyladipate ―2 | 2.00 |
| Propylparaben | 0.50 |
| Tocopherol | 0.10 |
| Disteardimonium Hectorite | 0.50 |
| Titanium Dioxide | 13.0 |
| Iron Oxide Yellow | 1.50 |
| Iron Oxide Red | 0.35 |
| Iron Oxides Brown | 1.00 |
| Cyclomethicone | 12.0 |
| Dimethicone/Vinyl Dimethicone Crosspolymer | 3.00 |
| Mica | 3.00 |
| TOTAL | 100.00 |

| Example 5 | |
|---|---|
| Ingredient | % weight |
| Isododecane | 57.0 |
| Cyclomethicone | 10.0 |
| Propylsilsesquioxane | 5.0 |
| Hydrogenated Polyisobutene | 7.0 |
| Polyisoprene | 3.5 |
| Iron Oxide Black | 7.0 |
| Sucrose Stearate | 1.5 |
| Disteardimonium Hectorite | 0.8 |
| Propylene carbonate | 0.2 |
| TOTAL | 100.0 |

| Example 6 | |
|---|---|
| Ingredient | % weight |
| Isododecane | 36.55 |
| Dimethicone | 20.00 |
| Dextrin Palpitate | 0.10 |
| Polyisoprene | 7.00 |
| Hydrogenated Vegetable Oil | 0.50 |
| Sorbitan Tristearate | 0.89 |
| Phytostearyl Isostearate | 0.50 |
| Propylparaben | 0.20 |
| Tocopherol | 0.10 |
| Ascorbyl Palmitate | 0.50 |
| Disteardimonium Hectorite | 3.50 |
| Propylene Carbonate | 1.16 |
| Mica and Titanium Dioxide | 10.00 |
| Iron Oxide | 10.00 |
| TOTAL | 100.00 |

| Example 7 | |
|---|---|
| Ingredient | % weight |
| Isododecane | 20.60 |
| Polyethylene | 23.00 |
| Cyclopentasiloxane and Polypropylsiloxane | 13.00 |
| Diisostearyl Malate | 3.00 |
| Polymethylsilsesquioxane | 3.50 |
| Polyisoprene | 8.00 |
| Propylparaben | 0.20 |
| Bht | 0.02 |
| Iron Oxide Brown | 6.20 |
| Iron Oxide | 5.40 |
| Iron Oxide Yellow | 0.75 |
| D&C Red 7 Ca Lake | 4.40 |
| Disteardimonium Hectorite | 0.30 |
| Propylene Carbonate | 0.10 |
| Glycerin | 1.03 |
| Coconut Oil and Tiare' Flower | 0.50 |
| TOTAL | 100.00 |

| Example 8 | |
|---|---|
| Ingredient | % weight |
| Isododecane | 22.95 |
| Polyethylene | 23.00 |
| Cyclopentasiloxane and Polypropylsiloxane | 13.00 |
| Diisostearyl Malate | 3.00 |
| Polymethylsilsesquioxane | 3.50 |
| Polyisoprene | 6.00 |
| Propylparaben | 0.20 |
| Bht | 0.02 |
| Mica and Titanium Dioxide | 10.20 |
| Iron Oxide | 3.40 |
| Iron Oxide Yellow | 0.50 |
| Titanium Dioxide | 1.50 |
| Disteardimonium Hectorite | 0.15 |
| Propylene Carbonate | 0.05 |
| Glycerin | 1.03 |
| Coconut Oil and Tiare' Flower | 0.50 |
| TOTAL | 100.00 |

| Example 9 | |
|---|---|
| Ingredient | % weight |
| Isododecane | 63.00 |
| Polyisoprene | 10.00 |
| Disteardimonium Hectorite | 8.50 |
| Propylene Carbonate | 2.00 |
| Alcohol | 0.80 |
| D&C Red 7 Ca Lake | 0.70 |
| Titanium Dioxide | 1.40 |
| Iron Oxide Yellow | 1.15 |
| Fd&C Blue 1 Al Lake | 0.50 |
| Iron Oxide Red | 0.85 |
| Silica | 0.30 |
| Acrylates/ethylhexyl acrylate copolymer | 8.00 |
| Mica and Titanium Dioxide | 2.60 |
| Flavour | 0.20 |
| TOTAL | 100.00 |

| Example 10 | |
|---|---|
| Ingredient | % weight |
| Isododecane | 65.00 |
| Polyisoprene | 10.00 |
| Disteardimonium Hectorite | 8.50 |
| Propylene Carbonate | 2.00 |
| Alcohol | 0.80 |
| D&C Red 7 Ca Lake | 0.70 |
| Titanium Dioxide | 1.40 |
| Iron Oxide Yellow | 1.15 |
| Fd&C Blue 1 Al Lake | 0.50 |
| Iron Oxide Red | 0.85 |
| Silica | 0.30 |
| Polyglyceryl-2 diisostearate/IPDI copolymer | 6.00 |
| Mica and Titanium Dioxide | 2.60 |
| Flavour | 0.20 |
| TOTAL | 100.00 |

## Claims

1. A polyisoprene-containing cosmetic composition, suitable for application to facial skin, lips and eyelashes, **characterized by** comprising :
from 2 to 25% of polyisoprene with a molecular weight between 100,000 and 4,000,000;
from 0.05 to 20% of oleophilic modified smectite clay;
from 1.1to 90% of organic solvent;
the balance comprising conventional cosmetic excipients, colourants and additives, all percentages being by weight of the final composition.

2. A composition according to claim 1, **characterized in that** polyisoprene has a molecular weight of between 2,000,000 and 4,000,000.

3. A composition according to claim 1 or 2, **characterized in that** the oleophilic modified clay is disteardimonium hectorite.

4. A composition according to anyone of claims 1-3, **characterized in that** the oleophilic modified clay is present in an amount of 0.1-10% .

5. A composition according to anyone of claims 1-4, **characterized in that** the organic solvent is an aliphatic hydrocarbon with 12-22 carbon atoms.

6. A composition according to claim 5, **characterized in that** the organic solvent is isododecane.

7. A composition according to anyone of claims 1-6, **characterized in that** the organic solvent is present in an amount of 10-80%.

8. A composition according to anyone of claims 1-7, **characterized in that** it further comprises from 0.5 to 20% of wax or silicone.

9. Use of a polyisoprene with a molecular weight of between 100,000 and 4,000,000 as film-forming agent in a cosmetic composition.

10. A method for preparing polyisoprene suitable for use in a composition according to anyone of claims 1-8, comprising comminuting a solid high molecular weight polyisoprene and mechanically degrading and depolymerizing the comminuted material in a high speed blade mixer to a lower molecular weight within the range of 100,000-4,000,000.
